# EUROPEAN PATENT SPECIFICATION

(11) **EP 0 535 874 B1**
(45) Date of publication and mention of the grant of the patent: **27.03.1996**
(21) Application number: 92308758.9
(22) Date of filing: 25.09.1992
(51) Int. Cl.: A61M 25/00

(54) **Co-axial catheter**
Koaxial-Katheter
Cathéter coaxial

(30) Priority: 26.09.1991 CA 2052300
(43) Date of publication of application: 07.04.1993
(73) Proprietor: MED-PRO DESIGN, INC., Mississauga, Ontario L5G 2M3 (CA)
(72) Inventor: Martin, Geoffrey, S., Mississauga, Ontario L5G 2M3 (CA)
(74) Representative: Bowles, Sharon Margaret

(56) References cited:
- EP-A- 0 025 704
- EP-A- 0 333 308
- US-A- 5 053 004

## Description

This invention relates to dual lumen catheters for use in haemodialysis treatments and more particularly to a dual lumen catheter having co-axial intake and return lumens.

Haemodialysis treatments have been developed since the early 1960s using a variety of combinations and arrangements of catheters. The earliest treatments were conducted using two needles in the same vein and this subsequently led to pioneer work done by Dr. Shaldon in England who used two flexible catheters which could be left in place for limited periods. It was recognized by some practioners that it would be preferable to use a single incision rather than to use two and this led to the development of techniques involving dual flow catheters. There are two basic types. The first to be attempted was a coaxial catheter with the intake lumen surrounding the return lumen. While this had advantages, there were some difficulties of manufacture. The other approach is to use side-by-side lumens either in individual tubes connected to one another or in a single tube divided by an interior septum so that the lumens are D-shaped. These structures also had advantages and disadvantages, the notable disadvantage being that because the lumens are side-by-side, the intake openings must be in one side of the catheter. As a consequence of this, if the catheter were to attach itself to the wall of a vein due to suction applied to the intake lumen, then of course the flow would stop. Medical staff then have to move the catheter by rotating it until blood again flows. This is a very delicate manipulation which is normally performed only by a qualified medical practioner who must be available at all times in case the flow is discontinued.

The side-by-side structures have advantages in manufacture due to the fact that the two lumens can be created simultaneously in an extrusion. This has led to great activity in developing devices having side-by-side D-shaped lumens at the expense of co-axial structures. Nevertheless, due to the inherent disadvantages of the side-by-side structures, there has been renewed interest in developing suitable co-axial devices. This is primarily because the intake lumen can have openings in any part of the wall of the catheter. As a result, no matter where the catheter may rest against a vein, some of the intake openings remain patent. There is then less likelihood that the procedure must be serviced by a trained medical practioner.

An early patent showing the use of co-axial tubing in haemodialysis treatments is U.S. Patent 4,037,599 to Raulerson. This structure involves the use of a needle forming the return lumen and a co-axial sleeve which is tapered at its leading end to follow the incision made by the needle and to provide an intake lumen between the tube and the needle. This structure can not of course be left in place, but it is an indication of the approach to the use of co-axial tubes to form two lumens

Another structure involving the use of a needle is found in U.S. Patent 4,073,297 to Kopp. Again this structure involves a tube about the needle to define an intake lumen and blood is returned through the needle.

A further approach to co-axial dual lumens is found in U.S. Patent 4,196,860 to McLaughlin. In this structure the intake lumen is open at its end and blood is withdrawn down this lumen about the return lumen. Further structures of a similar kind are to be found in U.S. Patent 4,202,332 to Tersteegen et al.

U.K. Patent Application GB 2017499 A teaches the use of a dual lumen co-axial catheter made of flexible plastics material. The inner tube projects beyond the outer tube to form a return lumen and the intake lumen is tapered at its end to close about the return lumen. This kind of structure is also to be found in Canadian Patent 1,150,122 to the present inventor.

A contrasting structure is shown in U.S. Patent 4,493,696 to Uldall. In this the outer tube converges to fit about the inner tube and then projects beyond the inner tube to form an extension of the inner tube at the tip. Although this contrasts with all of the previous structures mentioned, there are disadvantages in this arrangement, notably that there is a discontinuity where the end of the inner tube terminates, and the rigidity of the tip is determined by the material of the outer tube which of course must be sufficiently rigid to maintain its patency in use.

The most recent reference known to applicant involving a co-axial dual lumen arrangement is found in U.S. Patent 4,666,426 to Aigner which again uses the inner tube to form a tip.

These prior art structures suffer from contrasting design criteria which have the effect of limiting the acceptance of co-axial dual lumen catheters in haemodialysis. In all instances the tip section is made up of material formed from an extension of either the inner or outer tubes forming the dual flow portion of the catheter. As a result the tip section has the same physical characteristics as parts chosen to be sufficiently stiff to resist kinking if the catheter should be bent. By contrast, it is desirable that the tip section be sufficiently soft and pliable to permit this section to take up the local shape of a vein containing the catheter to avoid applying distorting forces to the vein and to permit prolonged access with a suitable selection of materials.

It is therefore among the objects of the present invention to provide an improved dual lumen co-axial catheter having a structure which permits the use of tips of softer material without affecting the rigidity of the main portion of the catheter.

Accordingly, in one of its aspects, the invention provides a co-axial dual lumen catheter of the type having a main section having proximal and distal ends and including an inner tube and an outer tube containing the inner tube, and combining with the inner tube to define an annular intake lumen, the outer tube extending along a longitudinal axis and defining intake openings providing access into the intake lumen;
a tubular tip section of smaller cross-section than the main section, the tip section having at least one return opening; and a transition portion between the main section and the tip section, the catheter being characterised by the transition portion comprising overlapping parts of the inner tube, the tip section, and the outer tube, the part of the inner tube being between the parts of the inner and outer tubes and the inner tube part blending into the tip section at the transition portion and the outer tube blending smoothly into the tip section with the intake lumen ending at the transition portion, the inner tube and the tip section forming a continuous outlet lumen projecting beyond the outer tube and the outlet lumen ending at said return opening.

In another of its aspects the invention provides a method of making a co-axial dual lumen catheter characterised by providing an outer tube of a first cross-section and having a selected length; providing an inner tube of a second cross-section proportioned to fit loosely within the outer tube leaving an annular passage between the tubes, the inner tube having a length comparable with said selected length; providing a short tubular tip section having a third cross-section proportioned to engage between the inner and outer tubes; positioning the inner tube inside the outer tube with an end part of the tip section engaged inside the outer tube and about the inner tube at a transition portion; applying heat and pressure to the transition portion while supporting the outer tube internally against collapse adjacent the transition portion, and providing internal support for the inner tube on the tip section through the transition portion to maintain a return lumen and to thereby form a blended transition portion defining a smooth outer surface; and forming at least one inlet opening in the outer tube adjacent the transition portion to provide for flow into an intake lumen defined by the space between the inner and outer tubes.

The invention will be better understood with reference to the drawings and following description, in which:
Fig. 1 is an isometric view of a catheter according to a preferred embodiment of the invention;
Fig. 2 is a sectional view of Fig. 1 drawn to a larger scale;
Figs. 3 and 4 illustrate steps in the procedure of manufacturing the catheter; and
Fig. 5 is a sectional view on line 5-5 of Fig. 1 and showing part of the catheter to a larger scale.

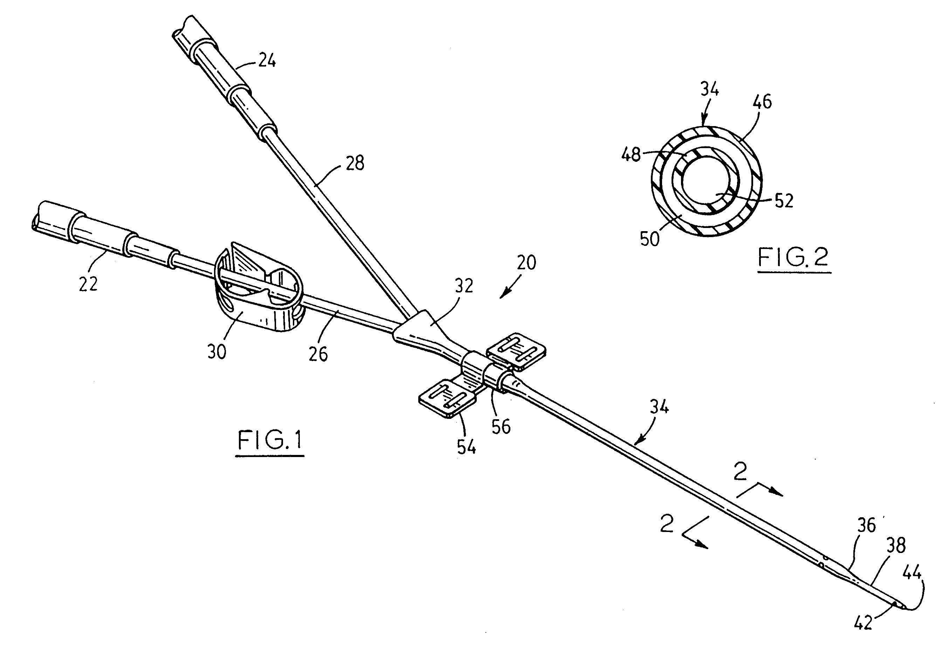

Reference is made firstly to Fig. 1 which illustrates a catheter designated generally by the numeral 20 and useful for withdrawing blood through an intake 22 and returning treated blood through an outlet 24. The intake and outlet are connected to flexible tubes 26, 28 which can be clamped using conventional devices such as device 30 shown on tube 26. The tubes meet at a junction 32 at the proximal end of a main section 34 which terminates at its distal end in a transition portion 36 leading to a tip section 38. Blood is withdrawn through side openings 40 and returns through further side openings 42 and end opening 44.

As seen in Fig. 2, the main section 34 includes an outer tube 46 containing an inner tube 48. The openings 40, shown in Fig. 1, supply blood to an intake lumen 50 formed between the tubes 46, 48 and blood returns by a return lumen 52 contained within the inner tube 48. Conventional connections are used at the proximal end of the main section 34 to connect to the tubes 26, 28 and the catheter is completed by provision of a wing structure 54 used to attach the catheter in place in conventional fashion. It is preferable that the wing structure be rotatable on the catheter and provision is made for this with location provided by a sleeve 56 which prevents movement of the catheter longitudinally relative to the wing structure.

The side openings 40 and 42 are typical of openings that can be provided around the periphery of the catheter to ensure flow into and out of the catheter from anywhere about the catheter. Consequently, if the catheter should be positioned so that some of the openings are occluded by positioning against the wall of a vein, other openings will take over and provide the essential flow.

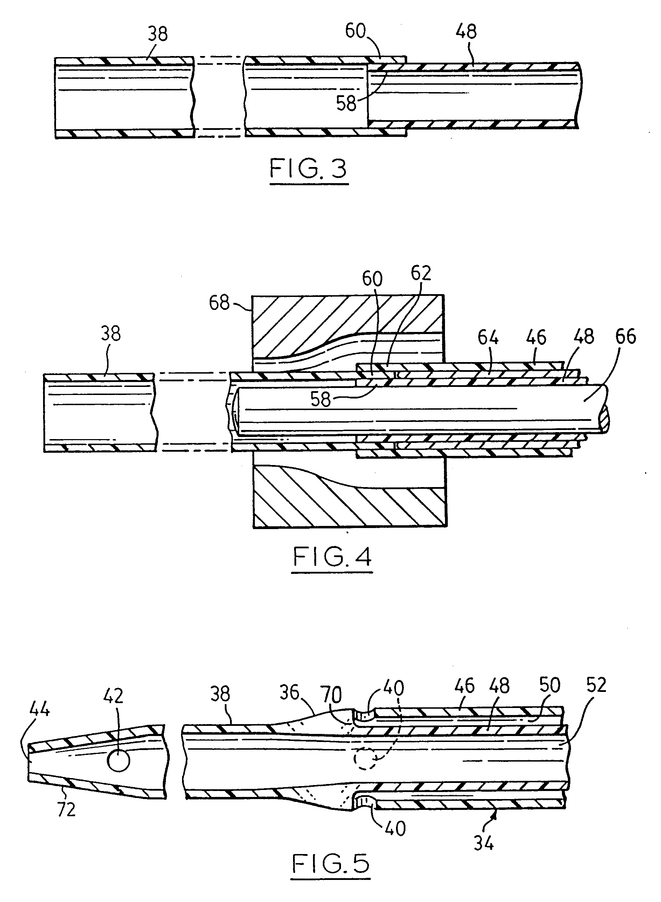

Reference is next made to Fig. 3 to illustrate a preliminary step in the manufacture of the catheter. As seen in Fig. 3, inner tube 48 has a leading part indicated by numeral 58 within a corresponding part 60 of the tip section 38. These parts can of course be deformed to fit together in this way, but as shown the round tubing is selected for these parts so that they fit within one another quite readily but at the same time quite closely. If preferred, the parts can be attached to one another using a suitable adhesive. Typically the inner tube is 6 French and the tip section 8 French. After this step has been completed, an assembly is then created as shown in Fig. 4. Here it will be seen that the outer tube 46 is now in place about the inner tube 48 and a leading part 62 of the outer tube overlaps part 60 of the tip section. Consequently the parts 58, 60 and 62 are located about one another and again an adhesive can be used to fix the assembly.

A tubular cylindrical mandrel 64 is proportioned to fit inside the outer tube 46 and about the inner tube 48. Typically the outer tube is 12 French and the materials of all of the inner and outer tubes and the tip section are polyurethane with the selection of the materials being chosen to give the physical characteristics desired. For instance if a soft tip is required, then a material of a suitable Durometer is provided for the tip section 38 and of course sufficent rigidity must be provided in the inner and outer tubes 46, 48 to ensure that they do not collapse under reasonable curvature needed to position the catheter completely on the patent. It should be noted that the inner tube is protected to some extent against collapse by the outer tube so that the inner tube can be of a relatively thin wall. This maximizes the space available for flow in the catheter.

A second mandrel 66 is provided to support the inner tube so that this tube extends between the mandrels 64 and 66. Mandrel 64 has a rounded end and stops against the part 60 of the tip section 38 whereas the inner mandrel 66 projects into the tip section 38. This provides support along the space occupied by two halves of a mould 68 which are operable to move into contact with the assembly.

The mould 68 is used to form the transition portion 36 by moving the mould halves into contact with the assembly shown in Fig. 4 under the influence of heat and pressure sufficient to cause the material of the parts 58, 60 and 62 to flow. Once this is completed the structure is allowed to cool and the mandrels removed. The result is shown in Fig. 5.

As seen in Fig. 5, the intake lumen 50 terminates at a blind annular end wall 70 at the transition portion 36. The intake lumen 50 is contained between outer tube 46 and inner tube 48 and the openings 40 are provided immediately adjacent the transition portion 36 to allow blood flow into the lumen 50. More openings can of course be provided further away from the transition portion if required.

The return lumen 52 is formed in part by the inner tube 48, the transition portion, and then blends into the tip section 38. Similarly, the transition portion provides blending smoothly from the outer surface of the tip section to the outer surface of the main section, and in particular to the outer surface of the outer tube 46.

It should be noted in Fig. 5 that the three parts, namely the outer and inner tubes 46, 48 and the tip section 38, are shown as three individual parts by the shading. Where they meet at the transition portion 36, the shading has been omitted because this is a portion where the materials flow into one another and it is indefinite where the parts begin and end after moulding. However by comparision between Figs. 4 and 5 it is evident that the parts 58, 60 and 62 form the material to create the transition portion 36. Preferably, these parts are all polyurethane with the grades being chosen to provide the desired physical characteristics such as a soft pliable tip section and a stiffer-main section.

After the assembly has been moulded as demonstrated in Figs. 4 and 5, the tip section 38 is deformed in a conventional manner to create a tapered tip 72 about the end opening 44. The combination of the tapered tip 72 and the transition portion 36 provides dilation of tissue as the catheter is moved over a Seldinger wire used during insertion and contained in the inner lumen 52 projecting through the end opening 44.

The catheter shown as a preferred embodiment is typical of catheters that could be made in accordance with the invention. As mentioned earlier it is possible to provide soft material for the tip to ensure that after insertion the tip will flex and will not damage veins. At the same time, there is sufficient rigidity in the transition portion to maintain the relationship between the tip and the inner and outer tubes so that the intake lumen 50 remains patent while the insertion takes place and during use. The arrangement is such that the lumen 50 is annular about the inner tube 48 right up to the intake openings 40 where the inner tube is anchored in the transition portion to ensure that all of the openings lead to a patent intake lumen.

Because the catheter is normally entered over a wire and into an opening created by a dilator, the soft tip section 38 will not be required to cause major dilation and consequently this section will withstand the relatively small force needed to push the tip section into the tissue. The final dilation takes place at the stronger transition portion which can withstand the force needed to give the final dilation to the size of the catheter.

It will be apparent that the structure can be varied within the scope of the invention. In particular, the tip section need not be tapered if insertion is through a sheath or by cut-down technique, and in such cases the distal end of the catheter could be closed. Also, the method of manufacture would be varied by extending the part 60 further over the inner tube 48 to a point where it is not affected by the forming of the transition portion. This unaffected end of part 60 would then naturally form the annular wall 70 without the use of the mandrel 64.

The proportions of the parts can of course be varied and it would be possible to do some preforming before assembly. For instance, if desired, the tip portion 38 could be made larger with the part 60 deformed inwardly to fit inside the inner tube 46. Conversely, the inner tube could be flared to fit around the tip section 38. Such variations are within the scope of the invention as described and claimed.

## Claims

1. A co-axial dual lumen catheter of the type having a main section (34) having proximal and distal ends and including an inner tube (48) and an outer tube (46) containing the inner tube, and combining with the inner tube to define an annular intake lumen (50), the outer tube extending along a longitudinal axis and defining intake openings (40) providing access into the intake lumen, a tubular tip section (38) of smaller cross-section than the main section (34), the tip section having at least one return opening (42,44); and a transition portion (36) between the main section (34) and the tip section (38), the catheter being characterised by the transition portion (36) comprising overlapping parts (58,60,62) of the inner tube, the tip section, and the outer tube, the part (60) of the inner tube being between the parts (58,62) of the inner and outer tubes and the inner tube part (58) blending into the tip section (38) at the transition portion and the outer tube (46) blending smoothly into the tip section (38) with the intake lumen (50) ending at the transition portion, the inner tube and the tip section forming a continuous outlet lumen projecting beyond the outer tube and the outlet lumen ending at said return opening.

2. A catheter as recited in claim 1 characterised in that the inner tube (48), outer tube (46) and tip section (38) are round in cross-section.

3. A catheter as recited in claim 2 characterised in that the tip section (38) is more flexible than the inner and outer tubes.

4. A catheter as recited in claims 2 or 3 characterised in that the diameter of the tip section (38) is greater than that of the inner tube (48) and less than that of the outer tube (46).

5. A catheter as recited in claims 1, 2, 3 or 4 characterised in that the return opening (44) is at a distal end of the catheter and the tip section (38) is tapered to converge towards said return opening.

6. A catheter as recited in claims 1, 2, 3, 4 or 5 characterised in that the tip section (38) includes an opening (44) at the distal end of the catheter.

7. A catheter as recited in claims 2, 3, 4, 5 or 6 characterised in that the outer tube (46) is about twice the diameter of the inner tube (48).

8. A catheter as recited in claims 2, 3, 4, 5, 6, 7 or 8 characterised in that the inner tube (48) and the outer tube (46) are spaced from one another radially about said axis by the part (60) of the tip section (38) at the transition portion (36).

9. A catheter as recited in claims 1, 2 or 3 characterised in that the inner tube (48) and the outer tube (46) are spaced from one another radially about said axis by a part (60) of the tip section (38) at the transition portion (36), and in which the transition portion is tapered smoothly to converge from the outer tube to the tip section.

10. A method of making a co-axial dual lumen catheter characterised by providing an outer tube (46) of a first cross-section and having a selected length; providing an inner tube (48) of a second cross-section proportioned to fit loosely within the outer tube leaving an annular passage between the tubes, the inner tube having a length comparable with said selected length; providing a short tubular tip section (38) having a third cross-section proportioned to engage between the inner and outer tubes; positioning the inner tube inside the outer tube with an end part of the tip section engaged inside the outer tube and about the inner tube at a transition portion (36); applying heat and pressure to the transition portion while supporting the outer tube internally against collapse adjacent the transition portion, and providing internal support for the inner tube on the tip section through the transition portion to maintain a return lumen and to thereby form a blended transition portion defining a smooth outer surface; and forming at least one inlet opening (40) in the outer tube adjacent the transition portion to provide for flow into an intake lumen (50) defined by the space between the inner and outer tubes.

## Patentansprüche

1. Koaxiales Doppelkanal-Katheter des Typs, bei dem ein Hauptteil (34) mit proximalen und distalen Endabschnitten und mit einem Innenrohr (48) und einem das Innenrohr umschließenden Außenrohr (46) vorhanden ist, wobei das Außenrohr gemeinsam mit dem Innenrohr einen ringförmigen Einlaßkanal (50) bildet, wobei sich das Außenrohr entlang einer Längsachse erstreckt und Einlaßöffnungen (40) festlegt, die einen Zugang in den Einlaßkanal bilden, mit einem rohrförmigen Kopfabschnitt (38), der einen kleineren Querschnitt als das Hauptteil (34) hat, wobei der Kopfabschnitt wenigstens eine Rücklauföffnung (42, 44) aufweist; und mit einem Übergangsbereich (36) zwischen dem Hauptteil (34) und dem Kopfabschnitt (38), dadurch gekennzeichnet, daß der Übergangsbereich (36) überlappende Teile (58, 60, 62) des Innenrohrs, des Kopfabschnitts und des Außenrohrs aufweist, wobei der Teil (60) des Innenrohrs zwischen den Teilen (58, 62) des Innenrohrs und des Außenrohrs liegt und der Teil (58) des Innenrohrs am Übergangsbereich in den Kopfabschnitt (38) übergeht und das Außenrohr (46) allmählich in den Kopfabschnitt (38) übergeht, wobei der Einlaßkanal (50) am Übergangsbereich endet, und wobei das Innenrohr und der Kopfabschnitt einen fortlaufenden Auslaßkanal bilden, der über das Außenrohr hinaus vorsteht, und der Auslaßkanal an der Rücklauföffnung endet.

2. Katheter nach Anspruch 1, dadurch gekennzeichnet, daß das Innenrohr (48), das Außenrohr (46) und der Kopfabschnitt (38) einen runden Querschnitt aufweisen.

3. Katheter nach Anspruch 2, dadurch gekennzeichnet, daß der Kopfabschnitt (38) flexibler als das Innenrohr und das Außenrohr ist.

4. Katheter nach Anspruch 2 oder 3, dadurch gekennzeichnet, daß der Durchmesser des Kopfabschnitts (38) größer als der des Innenrohrs (48) und kleiner als der des Außenrohrs (46) ist.

5. Katheter nach einem der Ansprüche 1 bis 4, dadurch gekennzeichnet, daß sich die Rücklauföffnung (44) an einem distalen Endabschnitt des Katheters befindet und sich der Kopfabschnitt (38) verjüngt, um auf die Rücklauföffnung hin konisch zuzulaufen.

6. Katheter nach einem der Ansprüche 1 bis 5, dadurch gekennzeichnet, daß der Kopfabschnitt (38) eine Öffnung (44) am distalen Endabschnitt des Katheters aufweist.

7. Katheter nach einem der Ansprüche 2 bis 6, dadurch gekennzeichnet, daß das Außenrohr (46) etwa den doppelten Durchmesser des Innenrohrs (48) aufweist.

8. Katheter nach einen der Ansprüche 2 bis 8, dadurch gekennzeichnet, daß das Innenrohr (48) und das Außenrohr (46) in radialer Richtung bezüglich der genannten Achse durch das Teil (60) des Kopfabschnitts (38) am Übergangsbereich (36) in einen gegenseitigen Abstand gebracht werden.

9. Katheter nach Anspruch 1, 2 oder 3, dadurch gekennzeichnet, daß das Innenrohr (48) gegenüber dem Außenrohr (46) in radialer Richtung bezüglich der genannten Achse durch ein Teil (60) des Kopfabschnitts (38) am Übergangsbereich (36) in einen gegenseitigen Abstand gebracht werden, wobei sich der Übergangsabschnitt allmählich verjüngt, um vom Außenrohr zum Kopfabschnitt konisch zuzulaufen.

10. Verfahren zum Herstellen eines koaxialen Doppelkanal-Katheters, gekennzeichnet durch folgende Schritte: Bereitstellen eines Außenrohrs (46) mit einem ersten Querschnitt und einer ausgewählten Länge; Bereitstellen eines Innenrohrs (48) mit einen zweiten Querschnitt, der so bemessen ist, daß das Innenrohr lose in das Außenrohr paßt und ein ringförmiger Durchlaß zwischen den Rohren verbleibt, wobei das Innenrohr eine Länge hat, die mit der genannten ausgewählten Länge vergleichbar ist; Bereitstellen eines kurzen, rohrförmigen Kopfabschnitts (38) mit einem dritten Querschnitt, der so bemessen ist, daß er zwischen das Innenrohr und das Außenrohr hineinpaßt; Positionieren des Innenrohrs innerhalb des Außenrohrs, wobei ein Endteil des Kopfabschnitts innerhalb des Außenrohrs und um das Innenrohr herum an einem Übergangsbereich (36) eingefügt ist; Aufbringen von Wärme und Druck auf den Übergangsbereich, während das Außenrohr benachbart zum Übergangsbereich von innen gegen Zusammendrücken abgestützt wird, und wobei das Innenrohr am Kopfabschnitt durch den Übergangsbereich hindurch von innen abgestützt wird, um einen Rücklaufkanal aufrechtzuerhalten und dadurch einen ineinander übergehenden Übergangsbereich auszubilden, der eine glatt verlaufende Außenfläche bildet; und Ausbilden wenigstens einer Einlaßöffnung (40) im Außenrohr benachbart zum Übergangsbereich, um eine Strömung in einen durch den Zwischenraum zwischen dem Innenrohr und dem Außenrohr gebildeten Einlaßkanal (50) zu ermöglichen.

## Revendications

1. Cathéter coaxial à double lumière du type comprenant une section principale (34) ayant des extrémités proximale et distale et comprenant un tube intérieur (48) et un tube extérieur (46) contenant le tube intérieur, et se combinant au tube intérieur pour définir une ouverture annulaire d'admission (50), le tube extérieur s'étendant le long d'un axe longitudinal et définissant des orifices d'admission (40) fournissant des accès dans l'ouverture d'admission, une section d'extrémité tubulaire (38) d'une section transversale plus petite que la section principale (34), la section d'extrémité ayant au moins un orifice de retour (42, 44); et une partie de transition (36) entre la section principale (34) et la section d'extrémité (38), le cathéter étant caractérisé par le fait que la section de transition (36) comprend des parties (58, 60, 62) recouvrant le tube intérieur, la section d'extrémité, et le tube extérieur, la partie (60) du tube intérieur étant entre les parties (58, 62) des tubes intérieur et extérieur et la partie de tube intérieur (58) imbriquée dans la section d'extrémité (38) au niveau de la section de transition, et le tube extérieur (46) étant imbriqué en douceur dans la section d'extrémité (38), avec l'ouverture d'admission (50) se terminant au niveau de la section de transition, le tube intérieur et la section d'extrémité formant une ouverture de sortie continue se projetant au-delà du tube extérieur et l'ouverture de sortie se terminant audit orifice de retour.

2. Cathéter selon la revendication 1, caractérisé en ce que le tube intérieur (48), le tube extérieur (46) et la section d'extrémité (38) présentent une section transversale ronde.

3. Cathéter selon la revendication 2, caractérisé en ce que la section d'extrémité (38) est plus flexible que les tubes intérieur et extérieur.

4. Cathéter selon l'une des revendications 2 ou 3, caractérisé en ce que le diamètre de la section d'extrémité (38) est plus grand que celui du tube intérieur (48) et plus petit que celui du tube extérieur (46).

5. Cathéter selon l'une des revendications 1, 2, 3 ou 4, caractérisé en ce que l'orifice de retour (44) est à une extrémité distale du cathéter et la section d'extrémité (38) est amincie pour converger vers ledit orifice de retour.

6. Cathéter selon l'une des revendications 1, 2, 3, 4 ou 5, caractérisé en ce que la section d'extrémité (38) comprend un orifice (44) à l'extrémité distale du cathéter.

7. Cathéter selon l'une des revendications 2, 3, 4, 5 ou 6, caractérisé en ce que le diamètre du tube extérieur (46) est égal à environ deux fois le diamètre du tube intérieur (48).

8. Cathéter selon l'une des revendications 2, 3, 4, 5, 6, 7 ou 8, caractérisé en ce que le tube intérieur (48) et le tube extérieur (46) sont espacés radialement l'un de l'autre autour dudit axe par la partie (60) de la section d'extrémité (38) au niveau de la section de transition (36).

9. Cathéter selon l'une des revendications 1, 2 ou 3, caractérisé en ce que le tube intérieur (48) et le tube extérieur (46) sont espacés radialement l'un de l'autre autour dudit axe par une partie (60) de la section d'extrémité (38) au niveau de la section de transition (36), et dans lequel la section de transition est amincie en pente douce pour converger du tube extérieur vers la section d'extrémité.

10. Procédé pour réaliser un cathéter coaxial à double ouverture, caractérisé en ce qu'il prévoit un tube extérieur (46) d'une première section transversale et ayant une longueur choisie; un tube intérieur (48) d'une seconde section transversale proportionnée pour s'adapter lâchement avec le tube extérieur laissant un passage annulaire entre les tubes, le tube intérieur ayant une longueur comparable à ladite longueur choisie; une section d'extrémité tubulaire courte (38) ayant une troisième section transversale proportionnée pour s'engager entre les tubes intérieur et extérieur; en ce qu'il prévoit le positionnement du tube intérieur à l'intérieur du tube extérieur avec une partie terminale de la section d'extrémité engagée à l'intérieur du tube extérieur et autour du tube intérieur au niveau d'une section de transition (36); en ce qu'il prévoit l'application d'une température et d'une pression à la section de transition tout en maintenant le tube extérieur intérieurement contre un écrasement adjacent à la section de transition, et un support intérieur pour le tube intérieur sur la section d'extrémité jusqu'à la section de transition pour maintenir une ouverture de retour et pour former de ce fait une section de transition mixte définissant une surface extérieure lisse; et en ce qu'il prévoit la formation d'au moins un orifice d'entrée (40) dans le tube extérieur adjacent à la section de transition pour fournir un flux dans une ouverture d'admission (50) définie par l'espace entre les tubes intérieur et extérieur.
